# EUROPEAN PATENT APPLICATION

(11) **EP 1 669 087 A1**
(43) Date of publication of application: **14.06.2006**
(21) Application number: 04773426.4
(22) Date of filing: 17.09.2004
(51) Int. Cl.: A61K 45/00, A61K 47/04, A61K 47/32, A61K 47/38, A61K 9/48, A61K 31/18

(54) **PREPARATION CONTAINING BASIC DRUG**

(30) Priority: 30.09.2003 JP 2003341247
(71) Applicant: Kyowa Pharmaceutical Inc. Co., Ltd., Osaka-shi, Osaka 5320011 (JP)
(72) Inventor: MURAI, Chizu, KYOWA PHARMACEUTICAL IND.CO., LTD., Sanda-shi, Hyogo 6691324 (JP)
(74) Representative: Daniels, Jeffrey Nicholas
(86) International application number: PCT/JP2004/014047
(87) International publication number: WO 2005/032588

(57) **Abstract**

The present invention provides a pharmaceutical preparation containing basic physiologically active substance and enteric polymer, which has improvements in releasability of the contained physiologically active substance in intestinal canal. The basic medicine-containing preparation of the present invention comprises one or more electrolytes by which the action of the enteric polymer to suppress release of the basic physiologically active substance is improved.

## Description

### TECHNICAL FIELD

The present invention relates to an oral pharmaceutical preparation with improvements in releasability of a physiologically active substance showing basicity (basic medicine).

### BACKGROUND ART

An orally administered physiologically active substance passes through the stomach in a strongly acidic environment to reach the small intestine where it is absorbed. However, a basic medicine having a high solubility in an acidic solution may be released all at once from the pharmaceutical preparation in the stomach in a strongly acidic environment. As a result, the concentration of the physiologically active substance in blood is rapidly increased after oral administration, which may result in occurrence of its side effect. Accordingly, a pharmaceutical preparation has been developed, of which component constitution is devised to permit release of the physiologically active substance to be regulated in digestive tract.

Such a pharmaceutical preparation includes, for example, a preparation wherein enteric polymer that is a polymer not dissolved in the stomach in an acidic environment but dissolved in the small intestine in neutral to basic environment is compounded as a medicine release-regulating agent. This enteric polymer has acidic groups such as carboxyl groups in the molecule, and is thus not dissolved in an acidic environment but dissolved in a basic environment. Accordingly, a pharmaceutical preparation compounded with enteric polymer is not disintegrated in the stomach, thus slightly releasing the medicine in the stomach but releasing the medicine in the small intestine in a neutral to basic environment. By further coating the surface of the preparation with enteric polymer, the release of the medicine in the stomach can further be prevented.

In addition, sustained release of the medicine can be exhibited in the small intestine by using enteric polymer. That is, enteric polymer is more easily disintegrated or dissolved in a more strongly alkaline environment, and the pH inside the small intestine is higher in a lower part (pH 6 in an upper part, and pH 7.6 or thereabout in middle to lower part). Therefore, the pharmaceutical preparation to which enteric polymer is added releases a larger amount of the medicine in a lower part of the small intestine than in a higher part having a higher chemical absorptivity. As a result, there is also the effect of higher stabilization of the concentration of the physiologically active substance in blood.

As such pharmaceutical preparation, for example, a preparation is disclosed in Japanese Examined Patent Publication No. Hei 7-72129. This preparation is produced by adding enteric polymer such as methacrylic acid copolymer to a mixture of physiologically active substance and crystalline cellulose, and then granulating the mixture.

As shown in Examples (Reference Example 1) described later, however, the present inventor revealed that when a physiologically active substance exhibits basicity, the release of such a basic medicine is significantly hindered by enteric polymer. That is, a pharmaceutical preparation containing basic physiologically active substance may fail to secure sufficient concentration of the medicine in blood when enteric polymer is added to the preparation.

### DISCLOSURE OF INVENTION

As described above, the technique of regulating the release of a physiologically active substance by adding enteric polymer is known. However, the enteric polymer has the problem of hindrance of the release of a physiologically active substance exhibiting basicity.

Accordingly, the problem to be solved by the present invention is to provide a pharmaceutical preparation containing basic physiologically active substance and enteric polymer, which has improvements in releasability of the contained physiologically active substance in the intestinal tract.

To solve the problem described above, the present inventor made extensive study on additive components capable of improving the substance releasability of a preparation comprising basic physiologically substance and enteric polymer. As a result, the inventor found that even if the enteric polymer is contained, excellent substance releasability can be secured by adding electrolyte such as calcium chloride, and completed the present invention.

The basic medicine-containing preparation according to the present invention comprises basic physiologically active substance, and further comprises one or more enteric polymers and one or more electrolytes.

In the basic medicine-containing preparation, it is preferable that the electrolyte is alkali metal salt and/or alkaline earth metal salt, and that the physiologically active substance is tamsulosin hydrochloride.

Preferably, the basic medicine-containing preparation of the invention consists of multilayer and has at least a central layer, an inside layer and an outside layer, wherein the inside layer contains the above-mentioned physiologically active substance and enteric polymer, the outside layer contains the enteric polymer as main component, and the above-mentioned electrolyte is contained in at least any one of the layers. In the preparation, it is preferable that a sparingly-solublebinder is further added to the inside layer. A capsular preparation, wherein the basic medicine-containing preparation is charged into capsule, is also a preferable embodiment.

### BEST MODE FOR CARRYING OUT THE INVENTION

The basic medicine-containing pharmaceutical preparation of the present invention exhibits excellent substance releasability, although containing both basic physiologically active substance and enteric polymer, and is thus extremely excellent as a pharmaceutical form of the physiologically active substance exhibiting basicity, such as tamsulosin hydrochloride.

Hereinafter, embodiments and effects of the present invention demonstrating the above characteristics are described in detail.

The essence of the basic medicine-containing preparation of the present invention is that the preparation comprises basic physiologically active substance, one or more enteric polymers and one or more electrolytes.

The "basic physiologically active substance" can be applied to the present invention as long as it exhibits basicity in general meaning, and is not particularly limited. Even if it is a physiologically active substance administered generally in the form of a salt, the salt can be added, as it is, to the pharmaceutical preparation of the present invention. Examples of the basic physiologically active substance include tamsulosin hydrochloride, that is, (-)-(R)-5-[2-[[2-(o-ethoxyphenoxy) ethyl]amino]propyl]-2-methoxybenzenesulfonamide hydrochloride.

The "enteric polymer" used in the present invention refers to a polymer having acidic residues in the molecule thereof to maintain the form of the preparation in the stomach in a strongly acidic environment but being dissolved in the small intestine in a weakly acidic to weakly basic environment to disintegrate the preparation to release the physiologically active substance. Examples of the enteric polymer include enteric celluloses such as hydroxypropylmethyl cellulose phthalate, hydroxypropylmethyl cellulose acetate succinate, carboxymethylethyl cellulose, cellulose acetate phthalate, hydroxypropylmethyl cellulose phthalate; enteric methacrylic acid copolymers such as methacrylic acid / methyl methacrylate copolymer, methacrylic acid / butyl acrylate copolymer, methacrylic acid / ethyl acrylate copolymer; enteric polyvinyls such as polyvinyl acetate phthalate, and one polymer may be selected therefrom, or two or more polymers are selected therefrom and used as a mixture thereof.

In the present invention, the "electrolyte" is not particularly limited as long as it is a medically acceptable inorganic electrolyte which can be highly dissolved in an aqueous solvent such as digestive fluids to release ions. As the electrolyte, alkali metal salt (such as sodium chloride, potassium chloride) and alkaline earth metal salt (such as magnesium chloride, calcium chloride) are preferable, and one electrolyte may be selected therefrom and used, or a mixture of two or more thereof may also be used.

As described above, the enteric polymer is dissolved or disintegrated mainly in the small intestine to exhibit an action of releasing the physiologically active substance. According to the present inventor' s finding, however, the enteric polymer having acidic residues tends to inhibit the release of basic physiologically active substance (see Reference Example 1 in the Examples described later). However, the releasability of the basic physiologically active substance can be improved by adding electrolyte to the preparation.

On the other hand, a preparation described in related art Japanese Examined Patent Publication No. Hei 7-72129 can also be constituted so as to contain basic physiologically active substance, enteric polymer and electrolyte. In this prior art publication, however, the preparation having such a constitution is not specifically disclosed. In addition, possibly due to a lack of experimental confirmation, the electrolyte in the publication ("metal alkyl halide or earth metal alkyl halide" in the publication) is recognized as component merely for "regulating the speed of release of the active substance", that is, as one for merely preventing the release of the physiologically active substance, and not recognized as one for improving the releasability of the physiologically active substance in the intestine as disclosed in the present invention.

The preparation form of the present invention is not particularly limited, and a general preparation form can be applied. The preparation is for example in the form of a uniform mixture of the constitutional components, a multilayer structure or a preparation coated with a film. In manufacturing the preparation, a core is used as a central layer on which a substance layer may be formed. The multilayer structure is for example a preparation having a core provided thereon a physiologically active substance-containing layer blended with physiologically active substance and enteric polymer, and coated thereon with a film mainly consisting of enteric polymer. Even if the electrolyte is incorporated into any of the porous core, the substance release regulation layer and the coating layer, its effect can be exhibited to achieve the object (see Examples 2-1 to 2-4).

The "core" referred to herein may be any material capable of serving as a seed for the physiologically active substance-containing layer laminated on the core, and may be electrolyte, polysaccharides (e.g. cellulose) or starch alone, or a mixture of sugar and starch. Preferably, granulated particles of electrolyte-containing crystalline cellulose are excellent as a core material, though the core is not particularly limited.

In the pharmaceutical preparation of the present invention, wax, fats, oils, glues and metal salts of aliphatic acids which can be generally added to the preparation may be used in addition to the materials described above.

The method of producing the basic medicine-containing preparation according to the present invention is not particularly limited; for example, a method is preferable where a physiologically active substance-containing layer comprising of a physiologically active substance alone or with at least one of filler additive, enteric binder, sparingly-soluble binder and the like is laminated on a core served as seed by a tumbling-layering method. The preparation thus produced may be laminated with a layer consisting of only enteric polymer or a composite layer consisting of enteric polymer and suitable additives (layer consisting of mainly enteric polymer) by a pharmaceutical manufacturing method.

In this case, the physiologically active substance-containing layer preferably contains, as components, physiologically active substance, sparingly-soluble binder such as ethyl cellulose, enteric binder such as hydroxypropylmethylcellulose phthalate,lactose,precipitated calcium carbonate, and starch, but is not limited thereto. In a preferable embodiment, sparingly-soluble binders such as ethyl cellulose among those described above are added.

Preferable materials of the layer consisting of mainly enteric polymer include plasticizers such as polyethylene glycol and monoacetyl fat in addition to the enteric polymer described above.

To regulate the speed of release of the substance, a method of changing the speed by selection of the type and amount of additive, as conducted in a known method, can be adopted. A physiologically active substance whose surface is modified by applying or coating hydrophobic or hydrophilic substance can also be used. For example, the hydrophobic substance is desirably a fat-soluble substance such as wax, fat, oil, lipophilic surfactant or the like, and the hydrophilic substance is desirably polyethylene glycol, propylene glycol, glycerin, hydrophilic surfactant or the like.

Production of unit preparation is conducted by tumbling including centrifugation, in a fluidized bed, or by a suitable combination thereof. Extruding granulation can also be applied as a method of producing the core and the physiologically active substance-containing layer all at once. The unit preparation of the present invention thus obtained is preferably 1 mm or less.

The basic medicine-containing preparation mentioned above is preferably charged into capsules, and the kind of capsules used is preferably hard capsules.

### Examples

Hereinafter, the present invention is described in more detail by reference to the Examples, but the scope of the present invention is not limited thereto.

### Reference Example 1

To examine the substance releasability of a preparation described in Japanese Examined Patent Publication No. Hei 7-72129 as prior art, the preparation was obtained by reference to Examples of this publication. That is, 200.0 g of crystalline cellulose and 0.4 g of tamsulosin hydrochloride were mixed for 3 minutes in a small high-speed propeller granulator. Separately, 40.0 g of milky emulsion (solids content, 12.0 g) consisting of methacrylic acid / ethyl acrylate copolymer and water was diluted with water so as to give 240 g of a mixture, then the mixture was added dropwise into the granulator to prepare small particles of about 0.5 mm. Then, the small particles were dried at 60°C for 6 hours in a tray dryer to give pharmaceutical preparation having tamsulosin hydrochloride, crystalline cellulose and enteric polymer mixed uniformly therein.

The resulting pharmaceutical preparation (53.1 mg) was examined in a dissolution test with water as test liquid at a paddle revolution at 50 rpm according to the dissolution test, Method 2 (Paddle method) described in B-679 to 695 in an explanatory booklet of The Japanese Pharmacopoeia, 14th ed. The amount of released tamsulosin was measured by chromatography. The operation conditions of liquid chromatography are as follows :
Detector: fluorescence detector (ex: 278 nm, em: 330 nm)
Column: Wakosil-II5C18 manufactured by Wako
internal diameter: about 4 mm
length: about 25 cm
Mobile phase: 0.01 M phosphate / acetonitrile (7 : 3)
Flow rate: 0.6 mL/min. (constant flow rate)

The dissolution rate was measured at suitable intervals under the conditions described above, and immediately after 360 minutes, 1 g of sodium chloride was added thereto. The measurement results are shown in Table 1.

**Table 1**

| Time (min) | 15 | 30 | 60 | 120 | 180 | 360 | Addition of NaCl |
|---|---|---|---|---|---|---|---|
| Dissolution rate (%) | 0 | 4.8 | 4.8 | 8.4 | 16.0 | 35.3 | 49.8 |

As described in above results, it was revealed that the dissolution rate of the preparation is low probably because the enteric polymer having carboxyl groups has an action of preventing the release of the basic substance. However, the amount of the released basic substance whose release was suppressed was increased all at once by adding electrolyte.

### Example 1

0.4 g of tamsulosin hydrochloride was dissolved in 40 g of milky emulsion consisting of 30% methacrylic acid / ethyl acrylate copolymer and water, and then water was added thereto so as to give 2400g of a mixture. Separately, 200 g of crystalline cellulose was tumbled as pharmaceutical cores, and then, 25 g of sodium chloride was added thereto. The milky emulsion was added dropwise thereinto to prepare small particles of about 0.5 mm, which were then dried at 60°C for 6 hours in a tray dryer to give a pharmaceutical preparation. A preparation not containing sodiumhydrochloride was also prepared as comparative example in the same manner as described above. The resulting preparations were examined in the dissolution test in the same manner as in Reference Example 1. The results are shown in Table 2.

**Table 2**

| Time (hr) | | 1 | 2 | 4 | 6 | 12 |
|---|---|---|---|---|---|---|
| Dissolution rate (%) | Control | 17.7 | 33.4 | 56.2 | 71.2 | 88.8 |
| | Addition of NaCl | 40.4 | 54.9 | 74.6 | 87.3 | 102.7 |

From the results described above, it was revealed that the release of the basic pharmaceutical preparation can be improved by adding electrolyte.

### Example 2-1

0.7 g of tamsulosin hydrochloride, 14 g of hydroxypropylmethyl cellulose phthalate and 42 g of ethyl cellulose were dissolved in a mixed solution of 945 g of ethanol and 105 g of water, and 21 g of corn starch, 28 g of talc and 39 g of calcium stearate were dispersed in the solution. Separately, 420 g of commercially available small particles of crystalline cellulose (diameter of 0.5 to 0.7 mm) were tumbled for use as cores, followed by adding dropwise the above mixture thereto to laminate the cores with a layer regulating the release of the physiologically active substance.

Then, 60 g of hydroxypropylmethyl cellulose phthalate and 6 g of monoacetin were dissolved in a mixed solution of 720 g of ethanol and 180 g of water, and 12 g of talc and 18 g of calcium stearate were dispersed therein to give a mixed solution. By using this mixed solution, the above particles laminated with the layer for regulating the release of the physiologically active substance was coated, whereby a preparation (preparation 2-1) was obtained.

### Example 2-2

420 g of small particles of crystalline cellulose (diameter of 0.5 to 0.7 mm) as cores were laminated with the layer for regulating the release of the physiologically active substance in the same manner as that of Example 2-1 except that 7 g of calcium chloride was added to the solution of tamsulosin hydrochloride. In addition, the layer for regulating the release of the physiologically active substance was laminated in the same manner as that of Example 2-1 to give a preparation containing calcium chloride in the physiologically active substance layer (Example 2-2).

### Example 2-3

350 g of commercially available small particles of crystalline cellulose (diameter of 0.5 to 0.7 mm) were impregnated with 245 g of aqueous solution containing 70 g of calcium chloride, and dried in a tray dryer at 60°C for 10 hours. 420 g of the thus prepared small crystalline cellulose particles containing calcium chloride were used as cores to prepare a preparation (preparation 2-3) in the same manner as that of Example 2-1.

### Example 2-4

350 g of commercially available small particles of crystalline cellulose (diameter of 0.5 to 0.7 mm) were coated with 245 g of aqueous solution containing 70 g calcium chloride while tumbling, and then dried in a tray dryer at 60°C for 10 hours. 420 g of the thus prepared small particles of crystalline cellulose coated with calcium chloride were used as cores to prepare a preparation (preparation 2-4) in the same manner as that of Example 2-1.

The preparations 2-1 to 2-4 obtained in Examples 2-1 to 2-4 were examined for changes with time in the released amount of tamsulosin hydrochloride in the same method as the dissolution test described in Example 1. The results are shown in Table 3.

**Table 3**

| Time (hr) | 2 | 4 | 8 | 12 |
|---|---|---|---|---|
| Preparation 2-1 (without electrolyte) | 0 | 2 | 8.5 | 15.5 |
| Preparation 2-2 (containing CaCl₂ in the physiologically active substance layer) | 15.2 | 31.5 | 53.4 | 66 |
| Preparation 2-3 (with the cores impregnated with CaCl₂) | 8.4 | 17.2 | 38.1 | 51.1 |
| Preparation 2-4 (with the cores coated with CaCl₂) | 10.7 | 27.5 | 45.9 | 59.5 |

From the above results, it was made evident that the basic substance releasability of the preparation not containing electrolyte (preparation 2-1) was suppressed, but the substance releasability of the preparations containing electrolyte in any one of the layers was significantly improved.

## Claims

1. A basic medicine-containing preparation **characterized in** comprising basic physiologically active substance, one or more enteric polymers and one or more electrolytes.

2. The basic medicine-containing preparation according to claim 1, wherein the electrolyte is alkali metal salt and/or alkaline earth metal salt.

3. The basic medicine-containing preparation according to claim 1 or 2, wherein the physiologically active substance is tamsulosin hydrochloride.

4. The basic medicine-containing preparation according to any one of claims 1 to 3, wherein:
consisting of multilayer containing at least a central layer, an inside layer and an outside layer,
the inside layer contains the physiologically active substance and the enteric polymer,
the outside layer contains the enteric polymer as main component, and
the electrolyte is contained in at least any one of the layers.

5. The basic medicine-containing preparation according to claim 4, wherein sparingly-soluble binder is further contained in the inside layer.

6. A capsular preparation, wherein the basic medicine-containing preparation according to any one of claims 1 to 5 is charged into a capsule.
